# EUROPÄISCHE PATENTANMELDUNG

(11) **EP 0 891 964 A1**
(43) Veröffentlichungstag der Anmeldung: **20.01.1999**
(21) Anmeldenummer: 98109634.0
(22) Anmeldetag: 27.05.1998
(51) Int. Cl.: C07C 67/03, C07C 69/157

(54) **Verfahren zur Herstellung von 3-Acetoxy-2-methylbenzoesäurechlorid**

(30) Priorität: 17.07.1997 DE 19730602
(71) Anmelder: Clariant GmbH, 65929 Frankfurt am Main (DE)
(72) Erfinder: Cosmo, Robert, Dr., 64291 Darmstadt (DE); Dierdorf, Andreas, Dr., 60529 Frankfurt (DE)

(57) **Zusammenfassung**

Die vorliegende Erfindung betrifft ein Verfahren zur Herstellung von 3-Acetoxy-2-methylbenzoesäurechlorid, indem man ein Alkalimetallsalz der 3-Aminonaphthalin-1,5-disulfonsäure mit Alkalimetallhydroxid und Wasser im Gewichtsverhältnis 1:(1 bis 1,6):(1 bis 1,6) bei 220 bis 320°C zum Dialkalisalz der 3-Hydroxy-2-methylbenzoesäure umsetzt, unlösliche Bestandteile aus dem Reaktionsgemisch abtrennt, das Reaktionsgemisch anschließend durch Zugabe von Säure auf einen pH-Wert von 11,5 bis 13,5 einstellt und bei -5 bis +25°C mit Acetanhydrid umsetzt, durch Zugabe von Säure die 3-Acetoxy-2-methylbenzoesäure ausfällt, die 3-Acetoxy-2-methylbenzoesäure abtrennt und mit einem anorganischen Säurechlorid zu 3-Acetoxy-2-methylbenzoesäurechlorid umsetzt.

## Beschreibung

Die vorliegende Erfindung betrifft ein vorteilhaftes Verfahren zur Herstellung von 3-Acetoxy-2-methylbenzoesäurechlorid.

3-Acetoxy-2-methylbenzoesäurechlorid stellt ein wichtiges Vorprodukt zur Herstellung pharmazeutischer Wirkstoffe dar. Insbesondere kann es als Baustein für neue hochwirksame HIV-Protease-Inhibitoren, die die biologische Aktivität des HIV-Protease-Enzyms hemmen, dienen. Durch die Hemmung der biologischen Aktivität des HIV-Protease-Enzyms wird die Vermehrung (Replikation) des HIV-Virus unterbunden. Auf diese Weise ergeben sich neue Behandlungsmöglichkeiten der Immunschwächeerkrankung Aids. Die Herstellung eines derartigen Wirkstoffes, der letztlich ein Decahydroisochinolinderivat darstellt, ist in der WO 95/09843 beschrieben. Wie aus Beispiel 82 der WO 95/09843 hervorgeht, setzt man [3S-(3R*, 4aR*, 8aR*, 2'S*, 3'R*)]-2-[3'Amino-2'-hydroxy-4'-phenyl]-butyl decahydroisochinolin-3-N-t-butylcarboxamid mit 3-Acetoxy-2-methylbenzoesäure in Gegenwart eines wasserentziehenden Mittels um. Die Umsetzung verläuft bei Raumtemperatur über einen Zeitraum von 2 Tagen und führt zur Ausbildung einer Amidbindung zwischen der 3-Acetoxy-2-methylbenzoesäure und der primären Aminogruppe des vorstehend genannten Decahydroisochinolins.

Nachteil dieser Synthese ist die recht lange Reaktionsdauer, die Verwendung eines wasserentziehenden Mittels sowie eine Ausbeute von lediglich 65 %.

Im Hinblick auf die vorangegangenen Darlegungen stellt sich die Aufgabe einen Stoff herzustellen, dessen Verwendung zu einer Vereinfachung der Synthese führt und der sich mit vertretbarem Aufwand auch in technischem Maßstab herstellen läßt.

Diese Aufgabe wird gelöst durch ein Verfahren zur Herstellung von 3-Acetoxy-2-methylbenzoesäurechlorid

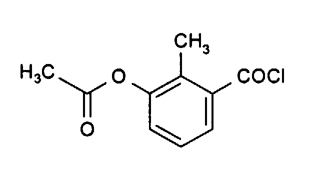

Die vorstehend erwähnten Nachteile können vermieden werden, indem man das vorstehend genannte Decahydroisochinolin mit 3-Acetoxy-2-methylbenzoesäurechlorid umsetzt. Die gute Reaktivität des 3-Acetoxy-2-methylbenzoesäurechlorids wird sich vorteilhaft in einer deutlichen Verkürzung der Reaktionszeit auswirken. Zudem kann auf die Verwendung eines wasserentziehenden Mittels, wie das in Beispiel 82 der WO 95/09843 verwendete Dicyclohexylcarbodiimid, ganz verzichtet werden.

Ein weiterer Vorteil besteht in der hohen Reinheit des 3-Acetoxy-2-methyl-benzoesäurechlorids, das sich einfacher und besser als die 3-Acetoxy-2-methylbenzoesäure reinigen läßt.

Während die 3-Acetoxy-2-methylbenzoesäure sich lediglich durch aufwendiges Umkristallisieren reinigen läßt, kann man das 3-Acetoxy-2-methylbenzoesäurechlorid mit geringem Aufwand durch fraktionierte Destillation reinigen.

Als zusätzlicher Vorteil ist zu erwähnen, daß die Produktverluste bei einer vergleichsweise einfachen fraktionierten Destillation niedriger als bei einer arbeitsintensiven Umkristallisation sind.

Gegenstand der vorliegenden Erfindung ist wie zuvor bereits erwähnt, ein Verfahren zur Herstellung von 3-Acetoxy-2-methylbenzoesäurechlorid.

In der WO 95/09843 ist ein mehrstufiges Verfahren zur Herstellung von 3-Acetoxy-2-methylbenzoesäure beschrieben. Dieses Verfahren geht von 3-Methoxybenzoylchlorid aus. Das 3-Methoxybenzoylchlorid wird mit Anilin zu 3-Methoxy-N-phenylbenzamid umgesetzt. In einem zweiten Schritt wird das 3-Methoxy-N-phenylbenzamid mit 2 Äquivalenten n-Butyllithium umgesetzt und anschließend mit Methyljodid alkyliert. Hierbei entsteht 3-Methoxy-2-methyl -N-phenylbenzamid, das anschließend mit wäßriger Salzsäure und wäßrigem Bromwasserstoff in siedender Essigsäure unter Verseifung der Amidgruppe und Spaltung der Methoxygruppe zur 3-Hydroxy-2-methylbenzoesäure umgesetzt wird. Die 3-Hydroxy-2-methylbenzoesäure muß mit Acetanhydrid acetyliert werden, um die 3-Acetoxy-2-methylbenzoesäure zu erhalten. Die einzelnen Reaktionsschritte sind in der WO 95/09843 unter Präparation 9 A, B und C und Beispiel 81 näher beschrieben.

Dieser Syntheseweg weist mehrere Nachteile auf. Zum einen muß die Umsetzung mit n-Butyllithium bei recht tiefen Temperaturen durchgeführt werden. Wie aus Präparation 9 B hervorgeht, liegen die Temperaturen im Bereich von -70 bis -15°C. Derartig tiefe Temperaturen lassen sich in technischem Maßstab nur mit sehr großem Aufwand realisieren.

Zum anderen ist der Gebrauch von Lithiumalkylen generell nicht unproblematisch und erfordert behutsamen und vorsichtigen Umgang mit dieser sehr reaktiven Stoffklasse. Die Handhabung in technischem Maßstab bedingt besondere Sicherheitsmaßnahmen und zusätzlichen apparativen Aufwand.

Ferner führt die Verwendung aggressiver Stoffe wie Salzsäure und Bromwasserstoff zu Werkstoffproblemen, da die Reaktionsapparaturen gegenüber diesem Stoff hinreichend korrosionsbeständig sein müssen. Das kann nur durch teure Werkstoffe erreicht werden.

Darüber hinaus ergeben sich Probleme bei der Entsorgung von mit Chlorwasserstoff und Bromwasserstoff belasteten Abgasen und Abwässern. Da weder Chlorwasserstoff noch Bromwasserstoff in die Umwelt gelangen dürfen, müssen sie aus den Abgasen und Abwässern gezielt entfernt werden.

Diese Nachteile vermeidet das erfindungsgemäße Verfahren zur Herstellung von 3-Acetoxy-2-methylbenzoesäurechlorid. Es ist dadurch gekennzeichnet, daß man ein Alkalimetallsalz der 3-Aminonaphthalin-1,5-disulfonsäure mit Alkalimetallhydroxid und Wasser im Gewichtsverhältnis 1:(1 bis 1,6):(1 bis 1,6) bei 220 bis 320°C zum Dialkalisalz der 3-Hydroxy-2-methylbenzoesäure umsetzt, unlösliche Bestandteile aus dem Reaktionsgemisch abtrennt, das Reaktionsgemisch anschließend durch Zugabe von Säure auf einen pH-Wert von 11,5 bis 13,5 einstellt und bei -5 bis 25°C mit Acetanhydrid umsetzt, durch Zugabe von Säure die 3-Acetoxy-2-methylbenzoesäure ausfällt, die 3-Acetoxy-2-methylbenzoesäure abtrennt und mit einem anorganischen Säurechlorid zu 3-Acetoxy-2-methylbenzoesäurechlorid umsetzt.

Zum leichteren Verständnis seien an dieser Stelle die Reaktionsschritte im nachfolgenden Reaktionsschema ausgehend vom Dinatriumsalz der 3-Aminonaphthalin-1,5-disulfonsäure als Alkalimetallsalz der 3-Aminonaphthalin-1,5-disulfonsäure (1) und Verwendung von Natriumhydroxid als Alkalimetallhydroxid - vereinfacht wiedergegeben.

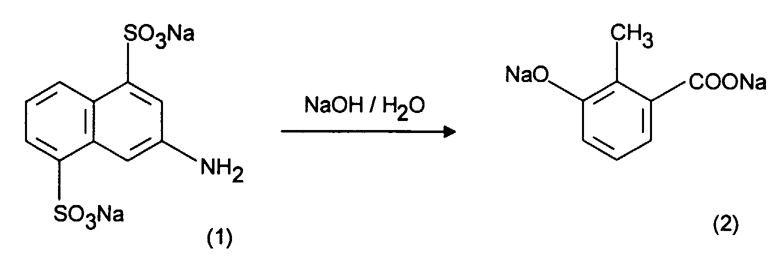

Das Dinatriumsalz der 3-Hydroxy-2-methylbenzoesäure (2) wird ohne Zwischenisolierung mit Acetanhydrid zu 3-Acetoxy-2-methylbenzoesäure (3) umgesetzt

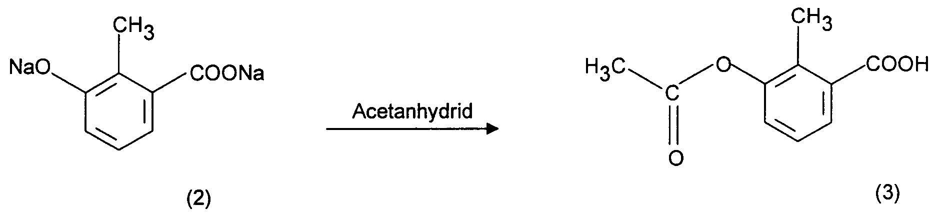

Die 3-Acetoxy-2-methylbenzoesäure (3) wird anschließend, beispielsweise mit Thionylchlorid, zu 3-Acetoxy-2-methylbenzoesäurechlorid (4) umgesetzt

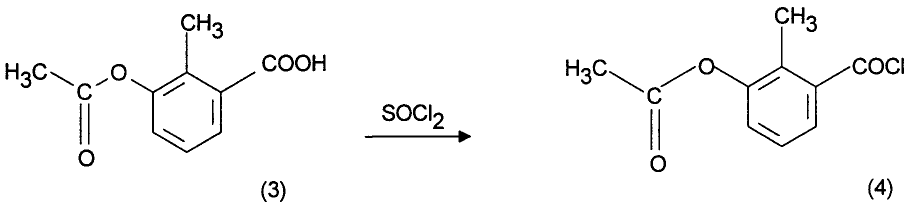

Die Vorteile des erfindungsgemäßen Verfahrens bestehen in einer kurzen Sequenz von Reaktionsschritten. Ferner kann auf eine Abtrennung respektive Isolierung des Alkalisalzes der 3-Hydroxy-2-methylbenzoesäure verzichtet werden und die Umsetzung mit Acetanhydrid läßt sich direkt mit dem von unlöslichen Bestandteilen befreiten und auf einem vorgegebenen pH-Wert eingestellten Reaktionsprodukt durchführen.

Es ist als überraschend anzusehen, daß sich die Umsetzung des Alkalisalzes der 3-Aminonaphthalin-1,5-disulfonsäure mit deutlich geringeren Mengen Alkalihydroxid und deutlich kürzeren Reaktionszeiten, als von Dean et al., J. Chem. Soc. [1961] Seite 2775 angegeben, durchführen läßt, ohne eine signifikante Minderung der Ausbeute in Kauf nehmen zu müssen.

Auf diese Weise läßt sich der Reaktor besser ausnutzen, da das Einsatzgemisch wesentlich mehr Alkalisalz der 3-Aminonaphthalin-1,5-disulfonsäure enthält und die Reaktionszeit kürzer ist. Zudem fallen weniger Abfallprodukte in Form von Salzen an und es wird auch weniger Säure zur Neutralisation der Alkalischmelze und Freisetzung der 3-Acetoxy-2-methylbenzoesäure benötigt.

Unter Alkalimetallsalz der Aminonaphthalin-1,5-disulfonsäure wird ein Monoalkalimetallsalz, ein Dialkalimetallsalz der 3-Aminonaphthalin-1,5-disulfonsäure oder ein Gemisch dieser Salze, insbesondere ein Monoalkalimetallsalz der 3-Aminonaphthalin-1,5-disulfonsäure verstanden.

Man setzt üblicherweise ein Mononatrium-, Monokalium-, Dinatrium-, Dikaliumsalz der 3- Aminonaphthalin-1,5-disulfonsäure oder ein Gemisch dieser Salze, insbesondere ein Mononatrium- oder Dinatriumsalz der 3-Aminonaphthalin-1,5-disulfonsäure oder ein Gemisch dieser Salze ein.

In vielen Fällen hat es sich bewährt, das Mononatriumsalz oder Dinatriumsalz, insbesondere das Mononatriumsalz der 3-Aminonaphthalin-1,5-disulfonsäure als Alkalimetallsalz der 3-Aminonaphthalin-1,5-disulfonsäure einzusetzen.

Man kann auch die freie 3-Aminonaphthalin-1,5-disulfonsäure einsetzen. Durch die Umsetzung mit Alkalimetallhydroxid bildet sich das entsprechende Alkalimetallsalz, welches das eigentliche Ausgangsmaterial zur Herstellung des Dialkalimetallsalzes der 3-Hydroxy-2-methylbenzoesäure darstellt.

Es hat sich bewährt, das Alkalimetallsalz der 3-Aminonaphthalin-1,5-disulfonsäure mit Alkalimetallhydroxid und Wasser im Gewichtsverhältnis 1:(1 bis 1,5):(1 bis 1,5), insbesondere 1:(1 bis 1,4):(1 bis 1,4) umzusetzen.

Man kann mit gutem Erfolg mit einem Überschuß an Alkalimetallhydroxid und Wasser arbeiten. In diesem Falle empfiehlt sich, das Alkalimetallsalz der 3-Aminonaphthalin-1,5-disulfonsäure mit Alkalimetallhydroxid und Wasser im Gewichtsverhältnis 1:(1 ,1 bis 1,5):(1,1 bis 1,5) insbesondere 1:(1,2 bis 1,4): (1,2 bis 1,4) umzusetzen.

Als Alkalimetallhydroxid setzt man Natriumhydroxid, Kaliumhydroxid oder ein Gemisch derselben, insbesondere Natriumhydroxid ein. Besonders einfach gestaltet sich das Verfahren, wenn man eine wäßrige Lösung des Alkalimetallhydroxids verwendet.

Man setzt das Alkalimetallsalz der 3-Aminonaphthalin-1,5-disulfonsäure, wie zuvor bereits erwähnt, bei 220 bis 320°C, insbesondere bei 250 bis 300, bevorzugt 260 bis 290°C mit dem Alkalihydroxid und Wasser zum Dialkalimetallsalz der 3-Hydroxy-2-methylbenzoesäure um.

Das Dialkalimetallsalz der 3-Hydroxy-2-methylbenzoesäure kann, je nachdem welches Alkalimetallsalz der 3-Aminonaphthalin-1,5-disulfonsäure und welches Alkalimetallhydroxid eingesetzt wurde, ein Dinatriumsalz, Dikaliumsalz oder ein beliebiges Gemisch von Natrium und Kalium enthaltenden Salzen der 3-Hydroxy-2-methylbenzoesäure sein.

Setzt man ein Natriumsalz als Alkalimetallsalz der 3-Aminonaphthalin-1,5-disulfonsäure und Natriumhydroxid als Alkalimetallhydroxid ein, so erhält man das Dinatriumsalz der 3-Hydroxy-2-methylbenzoesäure. Wie aus Formel (2) hervorgeht, ist das eine Natriumion der Carbonsäuregruppe und das andere dem Phenolatrest zuzuordnen.

Nach erfolgter Umsetzung läßt man das das Dialkalimetallsalz der 3-Hydroxy-2-methylbenzoesäure enthaltende Reaktionsgemisch abkühlen und trennt die unlöslichen Bestandteile bei einer Temperatur von 0 bis 50, insbesondere 15 bis 35°C ab.

Man kann die unlöslichen Bestandteile durch Zentrifugieren oder Filtrieren, insbesondere durch Filtrieren unter Verwendung eines Filterhilfsmittels abtrennen.

Unter Filterhilfsmittel werden chemisch indifferente, unlösliche Mittel, die zur Bildung eines lockeren, leicht abnehmbaren Filterkuchens führen und das Verstopfen des Filters oder des Filterkuchens oder das Durchlaufen feiner Niederschläge bei der Filtration verhindern, verstanden. Als Filterhilfsmittel können beispielsweise Asbest, Kieselgur, Cellulose und Cellulosederivate dienen.

Bei den unlöslichen Bestandteilen handelt es sich wahrscheinlich um anorganische Alkalisalze, die die nachfolgende Weiterverarbeitung ungünstig beeinflussen.

Das von den unlöslichen Bestandteilen befreite Reaktionsgemisch wird durch Zugabe von Säure, insbesondere durch Zugabe einer Mineralsäure auf einen pH-Wert von 11,5 bis 13,5, insbesondere 11,8 bis 13 eingestellt.

Man setzt Salzsäure, Essigsäure oder Schwefelsäure, insbesondere Salzsäure als Säure ein. Man kann die Säure in konzentrierter Form oder in Form einer verdünnten wäßrigen Lösung einsetzen.

In einer Vielzahl von Fällen hat es sich bewährt, das auf den gewünschten pH-Wert eingestellte Reaktionsgemisch bei 0 bis 20°C mit Acetanhydrid umzusetzen. Auf eine Abtrennung des Dialkalimetallsalzes der 3-Hydroxy-2-methylbenzoesäure oder der freigesetzten 3-Hydroxy-2-methylbenzoesäure kann verzichtet werden. Dadurch gestaltet sich das erfindungsgemäße Verfahren besonders einfach.

Man setzt je Mol Dialkalimetallsalz der 3-Hydroxy-2-methylbenzoesäure 1 bis 4, insbesondere 1,1 bis 3, bevorzugt 1,3 bis 2 Mol Acetanhydrid ein.

Üblicherweise legt man die wäßrige Lösung des Dialkalimetallsalzes der 3-Hydroxy-2-methylbenzoesäure vor und dosiert das Acetanhydrid unter Rühren sukzessive zu. Nach Ende der Zugabe läßt man noch eine gewisse Zeit nachreagieren, um die Umsetzung zu vervollständigen.

Während der Umsetzung hält man durch Kühlen die Temperatur auf dem vorgegebenen Wert.

Nach Beendigung der Umsetzung versetzt man das Reaktionsgemisch mit einer Säure und fällt die 3-Acetoxy-2-methylbenzoesäure aus. Man kann als Säure eine Mineralsäure, insbesondere Salzsäure verwenden. Im allgemeinen genügt es, die 3-Acetoxy-2-methylbenzoesäure bei einem pH-Wert von 1 bis 5, insbesondere 3 bis 4 auszufällen, indem man dem Reaktionsgemisch soviel Säure zusetzt, bis der gewünschte, im vorstehend genannten Bereich befindliche pH-Wert erreicht wird. Der die 3-Acetoxy-2-methylbenzoesäure enthaltende Niederschlag wird beispielsweise durch Zentrifugieren oder Filtrieren, insbesondere durch Filtrieren abgetrennt, mit Wasser gewaschen und das Wasser abgetrennt. Die 3-Acetoxy-2-methylbenzoesäure kann getrocknet werden und in trocknem Zustand weiterverarbeitet werden. Es ist allerdings auch möglich, die 3-Acetoxy-2-methylbenzoesäure in feuchtem Zustand mit dem anorganischen Säurechlorid umzusetzen. In diesem Fall liegt der Verbrauch an anorganischem Säurechlorid, das mit noch vorhandenem Wasserrest reagiert, höher als bei Verwendung einer getrockneten 3-Acetoxy-2-methylbenzoesäure.

Falls gewünscht, kann man die 3-Acetoxy-2-methylbenzoesäure, beispielsweise durch Umfällen oder Umkristallisieren, reinigen, bevor man sie mit dem anorganischen Säurechlorid umsetzt.

Es ist allerdings möglich, auf eine derartige zusätzliche Reinigung der 3-Acetoxy-2-methylbenzoesäure zu verzichten und statt dessen die rohe 3-Acetoxy-2-methylbenzoesäure in feuchtem oder trocknem Zustand weiterzuverarbeiten. Die 3-Acetoxy-2-methylbenzoesäure fällt als hinreichend reines Rohprodukt (Gehalt 95 bis 99,5 %, bestimmt durch HPLC-Analyse) an.

Durch die Verwendung von roher 3-Acetoxy-2-methylbenzoesäure gestaltet sich das erfindungsgemäße Verfahren besonders einfach, da auf eine separate Reinigung der 3-Acetoxy-2-methylbenzoesäure verzichtet werden kann. Aus diesem Grund wird üblicherweise dieser vorteilhaften Verfahrensvariante der Vorzug gegeben.

Man setzt die 3-Acetoxy-2-methylbenzoesäure bei 50 bis 100, insbesondere 70 bis 90°C in Anwesenheit oder Abwesenheit, insbesondere in Abwesenheit eines inerten Lösungsmittels mit dem anorganischen Säurechlorid um.

Als Lösungsmittel eignen sich beispielsweise Heptan, Diethylether, Cyclohexan, Toluol, o-Xylol, m-Xylol, p-Xylol, Gemische isomerer Xylole, Hexan. Es ist allerdings möglich, auf die Verwendung eines Lösungsmittels zu verzichten. Diese Variante wird, da sie besonders einfach ist, in der Regel bevorzugt angewendet.

Man setzt die 3-Acetoxy-2-methylbenzoesäure zu dem im anorganischen Säurechlorid vorhandenen, zum Austausch befähigten Chlor im Verhältnis 1:(1 bis 5), insbesondere 1:(1,5 bis 3), bevorzugt 1:(2 bis 2,5) ein.

Unter zum Austausch befähigtem Chlor versteht man das Chlor, das die Hydroxygruppe in der Carboxylgruppe der Carbonsäure substituiert und zur Bildung des entsprechenden Carbonsäurechlorids führt.

Man setzt als anorganisches Säurechlorid Thionylchlorid, Phosphortrichlorid oder Phosphorpentachlorid, insbesondere Thionylchlorid oder Phosphortrichlorid, bevorzugt Thionylchlorid ein. Thionylchlorid und Phosphorpentachlorid besitzen jeweils ein zum Austausch befähigtes Chlor. Demzufolge setzt man die 3-Acetoxy-2-methylbenzoesäure mit Thionylchlorid oder Phosphorpentachlorid üblicherweise im Molverhältnis 1:(1 bis 5), insbesondere 1:(1,5 bis 3), bevorzugt 1:(2 bis 2,5) um.

Phosphortrichlorid besitzt drei zum Austausch befähigte Chlorsubstituenten. Demzufolge setzt man die 3-Acetoxy-2-methylbenzoesäure und Phosphortrichlorid im Molverhältnis 1:(0,3 bis 1,5), insbesondere 1:(0,35 bis 1), bevorzugt 1:(0,4 bis 0,6) ein.

Nach Beendigung der Umsetzung destilliert man - soweit vorhanden - das überschüssige anorganische Säurechlorid ab. Das 3-Acetoxy-2-methylbenzoesäurechlorid, läßt sich durch fraktionierte Destillation unter reduziertem Druck aus dem Reaktionsgemisch - abhängig von den gewählten Destillationsbedingungen - in einer Reinheit von 98,5 bis 99,5 % gewinnen. Die Reinheit läßt sich, falls gewünscht, noch steigern, indem man beispielsweise das 3-Acetoxy-2-methylbenzoesäurechlorid nochmals fraktioniert destilliert.

Die destillativ abgetrennte anorganische Säurechlorid kann ebenso wie das gegebenenfalls abgetrennte Lösungsmittel wieder in die Reaktion eingesetzt werden.

Das erfindungsgemäße Verfahren läßt sich diskontinuierlich oder kontinuierlich, insbesondere diskontinuierlich durchführen.

Das nachfolgende Beispiel beschreibt die Erfindung näher, ohne sie zu beschränken.

### Experimenteller Teil

### Beispiel 1:

### 1a) Herstellung von 3-Acetoxy-2-methylbenzoesäure

In einem 500 ml Hastelloy-Autoklaven, der mit einem Propellerrührer ausgestattet ist, werden 100 g (0,31 mol) Monoatriumsalz der 3-Aminonaphthalin-1,5-disulfonsäure und 200 g einer 50 Gew.-% Natriumhydroxid-enthaltenden wäßrigen Lösung vorgelegt. Unter Rühren wird die aus den Einsatzstoffen entstehende Suspension 6 Stunden bei 280°C umgesetzt. Anschließend läßt man das Reaktionsgemisch auf 25°C abkühlen und spült es unter Zusatz von 100 ml Wasser aus dem Autoklaven.

Anschließend wird das Reaktionsgemisch unter Zugabe von 10 g eines Filterhilfsmittels auf Silikat-Basis filtriert, wodurch die unlöslichen Bestandteile aus dem Reaktionsgemisch abgetrennt werden.

Die Ausbeute an Dinatriumsalz der 3-Hydroxy-2-methylbenzoesäure beträgt 73 % (bestimmt durch HPLC-Analyse).

Durch Zugabe von konzentrierter Salzsäure wird ein pH-Wert von 12,5 eingestellt. Anschließend wird auf 0°C abgekühlt und unter Rühren werden zu der das Dinatriumsalz der 3-Hydroxy-2-methylbenzoesäure enthaltenden wäßrigen Lösung innerhalb von 15 Minuten 68,1 g (0,67 mol) Essigsäureanhydrid zugetropft. Man läßt unter Rühren 15 Minuten nachreagieren. Die Temperatur wird während der Acetanhydrid-Zugabe und während der Nachreaktion durch Kühlen bei 0°C gehalten.

Anschließend stellt man durch Zugabe von konzentrierter Salzsäure einen pH-Wert von 3,8 ein. Die Acetoxy-2-methylbenzoesäure fällt als Feststoff aus, wird abfiltriert, mit Wasser gewaschen und getrocknet. Man erhält 33,8 g 3-Acetoxy-2-methylbenzoesäure in einer Reinheit von 95 % (bestimmt durch HPLC-Analyse). Dies entspricht einer Ausbeute von 60 %, bezogen auf eingesetztes Mononatriumsalz der 3-Aminonaphthalin-1,5-disulfonsäure.

Falls die rohe 3-Acetoxy-2-methylbenzoesäure zusätzlich gereinigt werden soll, so kann man sie aus Essigsäureethylester oder Toluol umkristallisieren, wobei es sich empfiehlt, Aktivkohle zuzusetzen und die Lösung der 3-Acetoxy-2-methylbenzoesäure heiß zu filtrieren. Man erhält auf diese Weise 3-Acetoxy-2-methylbenzoesäure in einer Reinheit von 99,7 % (bestimmt durch HPLC-Analyse).

### 1b) Herstellung von 3-Acetoxy-2-methylbenzoesäurechlorid

Man legt in einem 250 ml Vierhalskolben, der mit Thermometer, Rückflußkühler und Magnetrührer bestückt ist, 31,1 g (0,16 mol) der rohen 3-Acetoxy-2-methylbenzoesäure (95 %ig wie in Beispiel 1 a erhalten) und 36,0 g (0,30 mol) Thionylchlorid vor. Man rührt und erhitzt bis zum Rückfluß. Die entstehenden Gase (HCl, SO₂) werden in wäßrige Natriumhydroxid-Lösung eingeleitet.

Wenn die Gasentwicklung nachläßt, destilliert man das überschüssige Thionylchlorid ab. Es kann für weitere Umsetzungen wieder verwendet werden. Der Rückstand wird über eine Destillationsbrücke bei 119 bis 122°C und 3 mbar destilliert. Man erhält 25,6 g 3-Acetoxy-2-methylbenzoesäurechlorid in einer Reinheit von 98,5 % (bestimmt durch GC-Analyse als 3-Acetoxy-2-methylbenzoesäuremethylester). Dies entspricht einer Ausbeute von 75,2 %, bezogen auf eingesetzte 3-Acetoxy-2-methylbenzoesäure.

### 3-Acetoxy-2-methylbenzoesäurechlorid:

- Fp.:: 35,2 °C
- MS: m/z: 214,212 [M⁺], 177 [M - Cl], 170 [M - CH₃CO], 135 ([M - Cl - CH₃CO], 100%)
- IR (KBr):/cm⁻¹: 3400 w, 3000 w, 1775 vs, 1440 m, 1370 m, 1210 vs, 1185 vs,
- ¹H-NMR:/ppm: (CDCl₃; 60 MHz)
δ = 2,33 (m, 6H, beide Methylgruppen)
δ = 7,1 - 7,5 (m, 2H, aromatische Protonen)
δ = 7,9 - 8,4 (m, 1H, aromatisches Proton)
- ¹³C-NMR:/ppm: (DMSO-d₆; 100 MHz)
δ =13; 20,5; 126; 127; 127,5; 131; 133; 149; 168; 169

## Patentansprüche

1. Verfahren zur Herstellung von 3-Acetoxy-2-methylbenzoesäurechlorid, dadurch gekennzeichnet, daß man ein Alkalimetallsalz der 3-Aminonaphthalin-1,5-disulfonsäure mit Alkalimetallhydroxid und Wasser im Gewichtsverhältnis 1:(1 bis 1,6):(1 bis 1,6) bei 220 bis 320°C zum Dialkalisalz der 3-Hydroxy-2-methylbenzoesäure umsetzt, unlösliche Bestandteile aus dem Reaktionsgemisch abtrennt, das Reaktionsgemisch anschließend durch Zugabe von Säure auf einen pH-Wert von 11,5 bis 13,5 einstellt und bei -5 bis 25°C mit Acetanhydrid umsetzt, durch Zugabe von Säure die 3-Acetoxy-2-methylbenzoesäure ausfällt, die 3-Acetoxy-2-methylbenzoesäure abtrennt und mit einem anorganischen Säurechlorid zu 3-Acetoxy-2-methylbenzoesäurechlorid umsetzt.

2. Verfahren nach Anspruch 1, dadurch gekennzeichnet, daß man das Alkalimetallsalz der 3-Aminonaphthalin-1,5-disulfonsäure mit Alkalimetallhydroxid und Wasser im Gewichtsverhältnis 1:(1 bis 1,5):(1 bis 1,5)umsetzt:.

3. Verfahren nach Anspruch 1 oder 2, dadurch gekennzeichnet, daß man als Alkalimetallhydroxid Natriumhydroxid, Kaliumhydroxid oder ein Gemisch derselben einsetzt.

4. Verfahren nach einem oder mehreren der Ansprüche 1 bis 3, dadurch gekennzeichnet, daß man als Alkalimetallhydroxid Natriumhydroxid einsetzt.

5. Verfahren nach einem oder mehreren der Ansprüche 1 bis 4, dadurch gekennzeichnet, daß man das Alkalimetallsalz der 3-Aminonaphthalin-1,5-disulfonsäure bei 250 bis 300°C umsetzt.

6. Verfahren nach einem oder mehreren der Ansprüche 1 bis 5, dadurch gekennzeichnet, daß man das Alkalimetallsalz der 3-Aminonaphthalin-1,5-disulfonsäure bei 260 bis 290°C umsetzt.

7. Verfahren nach einem oder mehreren der Ansprüche 1 bis 6, dadurch gekennzeichnet, daß man die unlöslichen Bestandteile bei 0 bis 50°C aus dem Reaktionsgemisch abtrennt.

8. Verfahren nach einem oder mehreren der Ansprüche 1 bis 7, dadurch gekennzeichnet, daß man die unlöslichen Bestandteile durch Zentrifugieren oder Filtrieren aus dem Reaktionsgemisch abtrennt.

9. Verfahren nach einem oder mehreren der Ansprüche 1 bis 8, dadurch gekennzeichnet, daß man das Reaktionsgemisch durch Zugabe einer Mineralsäure als Säure auf den pH-Wert von 11,5 bis 13,5 einstellt.

10. Verfahren nach einem oder mehreren der Ansprüche 1 bis 9, dadurch gekennzeichnet, daß man Salzsäure, Essigsäure oder Schwefelsäure als Säure einsetzt.

11. Verfahren nach einem oder mehreren der Ansprüche 1 bis 10, dadurch gekennzeichnet, daß man Salzsäure als Säure einsetzt.

12. Verfahren nach einem oder mehreren der Ansprüche 1 bis 11, dadurch gekennzeichnet, daß man bei 0 bis 20°C mit Acetanhydrid umsetzt.

13. Verfahren nach einem oder mehreren der Ansprüche 1 bis 12, dadurch gekennzeichnet, daß man je Mol Dialkalimetallsalz der 3-Hydroxy-2-methylbenzoesäure 1 bis 5 Mol Acetanhydrid einsetzt.

14. Verfahren nach einem oder mehreren der Ansprüche 1 bis 13, dadurch gekennzeichnet, daß man die 3-Acetoxy-2-methylbenzoesäure durch Zugabe einer Mineralsäure als Säure ausfällt.

15. Verfahren nach einem oder mehreren der Ansprüche 1 bis 14, dadurch gekennzeichnet, daß man die 3-Acetoxy-2-methylbenzoesäure bei einem pH-Wert von 1 bis 5, insbesondere 3 bis 4 ausfällt.

16. Verfahren nach einem oder mehreren der Ansprüche 1 bis 15, dadurch gekennzeichnet, daß man die 3-Acetoxy-2-methylbenzoesäure bei 50 bis 100, insbesondere 70 bis 90°C in Anwesenheit oder Abwesenheit eines inerten Lösungsmittels mit dem anorganischen Säurechlorid umsetzt.

17. Verfahren nach einem oder mehreren der Ansprüche 1 bis 16, dadurch gekennzeichnet, daß man die 3-Acetoxy-2-methylbenzoesäure zu dem im anorganischen Säurechlorid vorhandenen, zum Austausch befähigten Chlor im Verhältnis 1:(1 bis 5), insbesondere 1:(1,5 bis 3) einsetzt.

18. Verfahren nach einem oder mehreren der Ansprüche 1 bis 17, dadurch gekennzeichnet, daß man Thionylchlorid, Phosphortrichlorid oder Phosphorpentachlorid als anorganisches Säurechlorid einsetzt.
